# EUROPEAN PATENT SPECIFICATION

(11) **EP 3 384 932 B1**
(45) Date of publication and mention of the grant of the patent: **30.09.2026**
(21) Application number: 16870912.9
(22) Date of filing: 24.10.2016
(51) Int. Cl.: A61K 9/08, A61K 9/14, A61K 47/18, A61K 31/34, A61K 8/49, C07D 493/04, C07B 63/04

(54) **COMPOSITION FOR PRODUCING CONCENTRATED ANHYDROSUGAR ALCOHOL HAVING ENHANCED STABILITY, AND ANHYDROSUGAR ALCOHOL CONCENTRATION METHOD**
ZUSAMMENSETZUNG ZUR HERSTELLUNG VON KONZENTRIERTEM ANHYDROZUCKERALKOHOL MIT ERHÖHTER STABILITÄT UND ANHYDROZUCKERALKOHOLKONZENTRATIONSVERFAHREN
COMPOSITION POUR LA PRODUCTION DE POLYOL ANHYDRE CONCENTRÉ AYANT UNE STABILITÉ ACCRUE ET PROCÉDÉ DE CONCENTRATION DE POLYOL ANHYDRE

(30) Priority: 30.11.2015 KR 20150169247
(43) Date of publication of application: 10.10.2018
(73) Proprietor: Samyang Corporation, Seoul 03129 (KR)
(72) Inventor: RYU, Hoon, Daejeon 35235 (KR); LEE, Hyun Seung, Incheon 21376 (KR); YOO, Seung Hyun, Daejeon 34933 (KR); IM, Jun Seop, Daejeon 34186 (KR); JUNG, Young Jae, Daejeon 35215 (KR)
(74) Representative: Wohlfahrt, Jan Günther
(86) International application number: PCT/KR2016/011956
(87) International publication number: WO 2017/095016

(56) References cited:
- EP-A1- 3 384 933
- WO-A1-2014/178150
- JP-A- 2015 212 238
- JP-B2- 5 379 016
- KR-A- 20140 105 191
- KR-A- 20140 108 625
- KR-A- 20150 091 985
- KR-B1- 100 939 431

## Description

### [TECHNICAL FIELD]

The present invention relates to a composition for preparing concentrated anhydrosugar alcohol with improved stability and a method for concentrating anhydrosugar alcohol, and more specifically, to a composition comprising anhydrosugar alcohol and amine-based additive for preparing concentrated anhydrosugar alcohol with improved stability, and a method capable of providing anhydrosugar alcohol of good quality with low cost and high efficiency wherein concentration of anhydrosugar alcohol is conducted in the presence of amine-based additive, thereby the thermal stability of the anhydrosugar alcohol is improved so that the concentration process can be operated efficiently at high temperature, and at the same time, lowering of pH and UV transmittance of the anhydrosugar alcohol generated during the high temperature concentration process can be prevented.

### [BACKGROUND ART]

Hydrogenated sugar (also referred to as "sugar alcohol") means a compound obtained by adding hydrogen to the reductive end group in sugar, and generally has a chemical formula of HOCH₂(CHOH)ₙCH₂OH wherein n is an integer of 2 to 5. According to the number of carbon atoms, hydrogenated sugar is classified into tetritol, pentitol, hexitol and heptitol (4, 5, 6 and 7 carbon atoms, respectively). Among them, hexitol having 6 carbon atoms includes sorbitol, mannitol, iditol, galactitol, etc. and in particular, sorbitol and mannitol are very useful materials.

Anhydrosugar alcohol has a diol form with two hydroxyl groups in the molecule, and can be produced by using hexitol derived from starch (for example, Korean Patent No. 10-1079518 and Korean Laid-open Patent Publication No. 10-2012-0066904). Because anhydrosugar alcohol is an environmentally friendly material derived from recyclable natural resources, it has received much interest for a long time and researches on its production continue to proceed. Among such anhydrosugar alcohols, isosorbide produced from sorbitol has the widest industrial applicability at present.

Anhydrosugar alcohol can be used in various fields including treatment of heart and blood vessel diseases, patch adhesive, medicaments such as mouthwash, etc., solvents for compositions in the cosmetics industry, emulsifiers in the food industry, etc. In addition, it can increase the glass transition temperature of polymer materials like polyester, PET, polycarbonate, polyurethane, epoxy resin, etc., and improve the strength of such materials. Furthermore, because anhydrosugar alcohol is an environmentally friendly material derived from natural resources, it is very useful in the plastics industry such as bioplastics and the like. It is also known that anhydrosugar alcohol can be used as an adhesive, environmentally friendly plasticizer, biodegradable polymer, and environmentally friendly solvent for watersoluble lacquer. As such, anhydrosugar alcohol is receiving much interest because of its wide applicability, and the level of practical industrial application thereof is increasing.

Anhydrosugar alcohol after purification is concentrated to be prepared as a product in liquid or solid phase with a high concentration of 80% or higher. In order to concentrate it to such a high degree, the temperature must be raised up to 100°C or higher under vacuum condition. In such a case, however, thermal stability of the anhydrosugar alcohol deteriorates and thus UV transmittance of the final product is lowered. Accordingly, as an alternative, a method of concentrating anhydrosugar alcohol at a low temperature for a long time was suggested, but in this case, the concentration device becomes larger and the concentration becomes longer, resulting in increase of production cost and decrease of process efficiency.

Korean Patent No. 10-0939431 introduces a technique of adding a reducing agent such as NaBH₄ or an antioxidant such as butylated hydroxy toluene (BHT, 2,6-di-t-butyl-4-methylphenol) to distilled anhydrosugar alcohol, in order to improve stability of the anhydrosugar alcohol. However, the thermal stability of anhydrosugar alcohol is not improved sufficiently by this method, and thus it is difficult to prevent the UV transmittance lowering of anhydrosugar alcohol in high-temperature concentration. In addition, additive such as morpholine is not preferable since it smells bad and thus exerts bad influence on workability and product quality.

KR 10-2015-0091985 A relates to a method for purifying anhydrosugar alcohol comprising a step of mixing unpurified anhydrosugar alcohol and a hindered amine compound and a step of distilling the obtained mixture.

JP 5379016 B2 relates to improvement of the storage stability of anhydrosugar alcohol to be used as a raw material of a polymer in order to prevent quality degradation of the polymer due to lowering in reactivity of the anhydrosugar alcohol at the time of use and lowering in degree of polymerization of the polymer.

KR 10-0939431 B1 discloses the addition of a stabilizer before or after the concentration of anhydrosugar alcohol, wherein the purpose of the addition is to suppress generation of formic acid and mono-anhydro-hexose due to the decomposition and degradation of anhydrosugar alcohol during long term storage.

WO 2014/178150 A1 discloses a composition of concentrated anhydrosugar alcohol comprising a hindered amine compound for stabilization, wherein the composition can be obtained by distillation under reduced pressure and purification with active carbon.

JP 2015-212238 A discloses that anhydrosugar alcohol used as raw material in resin preparation has a relatively low stability and tends to decompose in the course of time. For this purpose, an amino alcohol having two active hydrogens is added to an anhydrosugar alcohol which was already purified and concentrated completely.

### [CONTENTS OF THE INVENTION]

### [PROBLEMS TO BE SOLVED]

The purpose of the present invention is to provide a composition for preparing concentrated anhydrosugar alcohol with improved stability, showing no lowering of pH and UV transmittance even in high-temperature concentration, and a method for concentrating anhydrosugar alcohol which can provide highly concentrated anhydrosugar alcohol of such a good quality with low cost and high efficiency.

### [TECHNICAL MEANS]

In one aspect, the present invention provides a composition for preparing concentrated anhydrosugar alcohol, comprising anhydrosugar alcohol, and amine compound as additive for stabilization, wherein the anhydrosugar alcohol is dianhydrohexitol, the amine compound is selected from piperidine, 1,8-diazabicycloundec-7-ene, 1,5-diazabicyclo[4.3.0]non-5-ene and combinations thereof, wherein the amine compound is unsubstituted or substituted by one or more substituents selected from linear or branched C₁-C₁₀alkyl, C₃-C₁₀cycloalkyl, aminoC₁-C₁₀alkyl, mono- or di-C₁-C₁₀alkylaminoC₁-C₁₀alkyl, carboxamide and amino, wherein the amount of the amine compound is 1 ppm or more and 300 ppm or less, based on the weight of the anhydrosugar alcohol, and wherein the concentrated anhydrosugar alcohol shows a water content of less than 1% by weight, and a transmittance of 90% or higher to ultraviolet (UV) light with 275 nm wavelength when diluted as an aqueous solution of 20% by weight concentration.

In another aspect, the present invention provides a method for concentrating anhydrosugar alcohol, wherein the concentration of anhydrosugar alcohol is conducted in the presence of amine compound as additive for stabilization, wherein the anhydrosugar alcohol is dianhydrohexitol, the amine compound is selected from piperidine, 1,8-diazabicycloundec-7-ene, 1,5-diazabicyclo[4.3.0]non-5-ene and combinations thereof, wherein the amine compound is unsubstituted or substituted by one or more substituents selected from linear or branched C₁-C₁₀alkyl, C₃-C₁₀cycloalkyl, aminoC₁-C₁₀alkyl, mono- or di-C₁-C₁₀alkylaminoC₁-C₁₀alkyl, carboxamide and amino, wherein the amount of the amine compound is 1 ppm or more and 300 ppm or less, based on the weight of the anhydrosugar alcohol, wherein the concentration is conducted at a temperature of 90°C to 110°C under a pressure condition of 10 mmHg (= 1.33322 kPa) to 100 mmHg (= 13.3322 kPa) for 30 minutes or longer, and wherein the anhydrosugar alcohol concentrated by the method shows a water content of less than 1% by weight, and a transmittance of 90% or higher to ultraviolet (UV) light with 275 nm wavelength when diluted as an aqueous solution of 20% by weight concentration.

The present disclosure further refers to an anhydrosugar alcohol concentrated according to the above method, showing a transmittance of 90% or higher to ultraviolet (UV) light with 275 nm wavelength when diluted as an aqueous solution of 20% by weight concentration.

### [EFFECT OF THE INVENTION]

According to the present invention, highly concentrated anhydrosugar alcohol, which shows water content of less than 1%, a UV transmittance of 90% or higher (with regard to 275 nm wavelength) and a stable pH of 6.5 to 7.5 when diluted as an aqueous solution of 20% by weight concentration, can be obtained easily with low cost and high efficiency.

### [CONCRETE MODE FOR CARRYING OUT THE INVENTION]

The present invention is explained in more detail below.

In the present invention, 'hydrogenated sugar' is generally also referred to as 'sugar alcohol' and it means a compound obtained by adding hydrogen to the reductive end group in sugar. Hydrogenated sugar is classified according to the number of carbon atoms into tetritol, pentitol, hexitol and heptitol (4, 5, 6 and 7 carbon atoms, respectively). Among them, hexitol having 6 carbon atoms includes sorbitol, mannitol, iditol, galactitol, etc. and in particular, sorbitol and mannitol are very useful materials.

In the present invention, 'anhydrosugar alcohol' means any material that is obtained by removing one or more water molecules from the original structure of the hydrogenated sugar in any manner in one or more steps.

In the present invention, hexitol is used as the hydrogenated sugar, and preferably, hydrogenated sugar selected from sorbitol, mannitol, iditol or mixtures thereof is used, and more preferably, sorbitol, which can be prepared easily through hydrogenation reaction of glucose derived from starch, is used.

In the present invention, the anhydrosugar alcohol is dianhydrohexitol which is the dehydrated product of hexitol, and preferably, the anhydrosugar alcohol is selected from isosorbide (1,4-3,6-dianhydrosorbitol), isomannide (1,4-3,6-dianhydromannitol), isoidide (1,4-3,6-dianhydroiditol) or mixtures thereof. Among them, isosorbide is particularly useful for industrial application.

The amine compound used as additive for stabilization in the present invention is selected from piperidine, 1,8-diazabicycloundec-7-ene (DBU), 1,5-diazabicyclo[4.3.0]non-5-ene (DBN) and combinations thereof, wherein the amine compound is unsubstituted or substituted by one or more (e.g., 1 to 4) substituents selected from linear or branched C₁-C₁₀alkyl, C₃-C₁₀cycloalkyl, aminoC₁-C₁₀alkyl, mono- or di-C₁-C₁₀alkylaminoC₁-C₁₀alkyl, carboxamide or amino.

In an embodiment, the amine compound may have a pKa of 8 to 15.

The amount of the amine compound as additive for stabilization in the composition for preparing concentrated anhydrosugar alcohol of the present invention, based on the weight of the anhydrosugar alcohol, is 1 ppm or more, preferably 2 ppm or more, preferably 5 ppm or more, preferably 8 ppm or more, or preferably 10 ppm or more, and is 300 ppm or less, preferably 200 ppm or less, preferably 100 ppm or less, preferably 80 ppm or less, or preferably 60 ppm or less.

The composition for preparing concentrated anhydrosugar alcohol of the present invention shows a transmittance of 90% or higher (more preferably, 94% or higher) to ultraviolet (UV) light with 275 nm wavelength when diluted as an aqueous solution of 20% by weight concentration.

In addition, the composition for preparing concentrated anhydrosugar alcohol of the present invention shows a water content of less than 1% by weight (more preferably, less than 0.1% by weight). The solid content of the composition may be 60% by weight or more (e.g., 60% by weight to 99.9% by weight), and preferably 70% by weight or more (e.g., 70% by weight to 99.9% by weight).

In addition, the composition for preparing concentrated anhydrosugar alcohol of the present invention may show a stable pH of 6.5 to 7.5 (e.g., measured at room temperature (25±3°C)) when diluted as an aqueous solution of 20% by weight concentration.

In addition, the composition for preparing concentrated anhydrosugar alcohol of the present invention may be in liquid or solid form, and more concretely, in aqueous solution from (for example, aqueous solution with anhydrosugar alcohol concentration of 60% by weight or higher), pellet form, chip form or flake form, but it is not limited thereto.

The method for concentrating anhydrosugar alcohol of the present invention is characterized in conducting the concentration of anhydrosugar alcohol in the presence of the amine compound explained above as additive for stabilization, for example, in the amount explained above.

In the method for concentrating anhydrosugar alcohol according to the present invention, the concentration is conducted at a temperature of 90°C to 110°C under a pressure condition of 10 mmHg (= 1.33322 kPa) to 100 mmHg (= 13.3322 kPa) for 30 minutes or longer (e.g., 30 minutes to 4 hours). The concentration may be conducted in a conventional concentration device (for example, rotary evaporator, forced circulation evaporator, thin film evaporator).

The anhydrosugar alcohol concentrated by the method of the present invention is dianhydrohexitol. According to an embodiment, the anhydrosugar alcohol concentrated by the method of the present invention may be that prepared by dehydration reaction of hydrogenated sugar.

There is no special limitation to the method of dehydrating hydrogenated sugar, and any method conventionally known in this field of art can be used as it is or with proper modification.

In dehydrating hydrogenated sugar and converting it to anhydrosugar alcohol, an acid catalyst is preferably used.

According to an embodiment, as the acid catalyst, one or more selected from the group consisting of sulfuric acid, nitric acid, hydrochloric acid, phosphoric acid, p-toluenesulfonic acid, methanesulfonic acid, ethanesulfonic acid, benzenesulfonic acid, naphthalenesulfonic acid or aluminum sulfate can be used, and preferably, sulfuric acid and other acid (for example, p-toluenesulfonic acid, methanesulfonic acid, ethanesulfonic acid, benzenesulfonic acid, naphthalenesulfonic acid or aluminum sulfate) can be mixed and used. The use amount of acid catalyst is preferably 0.5 to 10 parts by weight, based on 100 parts by weight of hydrogenated sugar (for example, hexitol).

The dehydration reaction of hydrogenated sugar may be conducted in the presence of acid catalyst as explained above at a temperature condition of 105°C to 190°C under a pressure condition of 1 mmHg (= 0.133322 kPa) to 100 mmHg (= 13.3322 kPa) for 1 hour to 10 hours), but it is not limited thereto.

In case of using acid catalyst in the dehydration reaction of hydrogenated sugar, the reaction product liquid is preferably neutralized with known alkali such as sodium hydroxide. The pH of the neutralized reaction product liquid is preferably 6 to 8. In addition, the product liquid of the dehydration reaction of hydrogenated sugar may be pre-treated under heating/reduced pressure before feeding it into the subsequent treatment step (for example, distilling step) in order to remove moisture and a low-boiling-point substance(s) remaining in the dehydration reaction product liquid.

Preferably, the anhydrosugar alcohol concentrated by the method of the present invention is that obtained by distilling the dehydration reaction product liquid resulting from the dehydration reaction of hydrogenated sugar as explained above, and then purifying the distillation product.

In an embodiment, the distillation may be conducted at a temperature condition of preferably from 100°C to 250°C, more preferably from 100°C to 200°C, and still more preferably from 110°C to 170°C, and under a pressure condition of preferably 10 mmHg (= 1.33322 kPa) or less (e.g., 0.0001 to 10 mmHg (= 0.0133322 Pa to 1.33322 kPa), more concretely 0.0001 to 8 mmHg (= 0.0133322 Pa to 1.066576 kPa)), more preferably 5 mmHg (= 0.66661 kPa) or less (e.g., 0.001 to 5 mmHg (= 0.133322 Pa to 0.66661 kPa)), and still more preferably 1 mmHg (= 0.133322 kPa) or less (e.g., 0.01 to 1 mmHg (= 1.33322 Pa to 0.133322 kPa), more concretely 0.01 to 0.8 mmHg (= 1.33322 Pa to 0.1066576 kPa)). If necessary, the distillation may be conducted through two or more steps. There is no special limitation to the method and device for the distillation, and any method and device conventionally known in this field of art may be utilized as it is or with proper modification. For example, a general condenser-type evaporator or column distillator may be used, or a thin-film evaporator may be utilized for the distillation.

In an embodiment, the purification may be conducted by one or more processes selected from crystallization, decolorization, cation exchange resin treatment or anion exchange resin treatment. In a purification process of a preferable embodiment, crystallization of the distillation product, decolorization of the crystallization product, and cation exchange resin treatment followed by anion exchange resin treatment of the decolorization product can be conducted subsequently.

There is no special limitation to the method and device for the crystallization, and any crystallization method and device conventionally known in this field of art may be utilized as it is or with proper modification. For example, concretely, it is possible to use a method of dissolving anhydrosugar alcohol in a solvent such as water, ethyl acetate, acetone, toluene, benzene, xylene, alcohol, etc. at an elevated temperature if necessary, and then lowering the temperature of the solution to precipitate the anhydrosugar alcohol crystals, or a method of melt crystallization using no solvent may be used.

The decolorization can be conducted by contacting an aqueous solution, where the crystallite of anhydrosugar alcohol is dissolved in water (for example, distilled water), with active carbon. As the active carbon, one or more selected from active carbon groups obtained by activating a plant source such as wooden material, palm, etc. or a mineral source such as brown coal, bituminous coal, soft coal, anthracite coal, etc. may be used. The average particle size of the active carbon is preferably from 0.25 to 1.0 mm, and more preferably from 0.25 mm to 0.70 mm. There is no special limitation in the manner of contacting the aqueous solution of anhydrosugar alcohol with active carbon. For example, the contact may be conducted in a manner of passing the aqueous solution of anhydrosugar alcohol through a column packed with the active carbon, or it may alternatively be conducted in a manner of incorporating the aqueous solution of anhydrosugar alcohol and the active carbon into a reactor and mixing them with agitation for a given time.

The cation exchange resin treatment may be accomplished by contacting the decolorization product liquid with cation exchange resin, and this may be conducted in a manner of passing the decolorization product liquid through a column packed with cation exchange resin. As the cation exchange resin, all of strong cation exchange resin (e.g., TRILITE-SCR-B) and weak cation exchange resin (e.g., DIAION WK11) may be used, and strong cation exchange resin is preferably used. As the strong cation exchange resin, one or more selected from H-form strong cation exchange resin (e.g., TRILITE-SCR-BH) or Na-form strong cation exchange resin (e.g., TRILITE-SCR-B) may be used preferably.

The anion exchange resin treatment may be conducted in a manner of passing the cation exchange resin treatment product liquid through a column packed with anion exchange resin. As the anion exchange resin, all of strong anion exchange resin (e.g., TRILITE AMP24) and weak anion exchange resin (e.g., DIAION WA10) may be used, and strong anion exchange resin is preferably used. As the strong anion exchange resin, Cl-form strong anion exchange resin (e.g., TRILITE AMP24) may be used preferably.

If the anhydrosugar alcohol prepared and purified as explained above is concentrated according to the method of the present invention, highly concentrated anhydrosugar alcohol showing good UV transmittance can be obtained.

The anhydrosugar alcohol concentrated by the method according to the present invention shows a water content of less than 1% by weight (more preferably, less than 0.5% by weight), and a transmittance of 90% or higher (more preferably, 94% or higher) to ultraviolet (UV) light with 275 nm wavelength when diluted as an aqueous solution of 20% by weight concentration.

In addition, the anhydrosugar alcohol concentrated according to the present invention may show a stable pH of 6.5 to 7.5 (e.g., measured at room temperature (25±3°C)) when diluted as an aqueous solution of 20% by weight concentration.

In addition, the anhydrosugar alcohol concentrated according to the present invention may be in liquid or solid form, and more concretely, in aqueous solution from (for example, aqueous solution with anhydrosugar alcohol concentration of 60% by weight or higher), pellet form, chip form or flake form, but it is not limited thereto.

The present invention is explained in more detail through the following Examples and Comparative Examples. However, the Examples are intended to facilitate understanding of the present invention only, and the scope of the present invention is not limited thereby.

### [EXAMPLES and COMPARATIVE EXAMPLES]

### Preparation Example

1,200 g of sorbitol powder (D-sorbitol, Samyang Genex Inc.) was fed into a four-neck glass reactor equipped with an agitator and melted by heating to 110°C. 12 g of concentrated sulfuric acid (Duksan Chemical, 95%) and 7.2 g of methanesulfonic acid (Sigma, 70%) were added thereto, and the reaction mixture was heated to 135°C. In maintaining this temperature, dehydration reaction was conducted for 4 hours under a vacuum condition of 40 torr (= 5.33288 kPa) to convert the starting material, sorbitol, to the anhydrosugar alcohol, isosorbide. After the dehydration reaction, the reaction mixture was cooled to 110°C, and 31.2 g of 50% sodium hydroxide solution (Samjeon Pure Chemical) was added thereto for neutralization.

The neutralized anhydrosugar alcohol was distilled by using a thin-film evaporator at 180°C under vacuum of 5 mmHg (= 0.66661 kPa) or less. The purity of the obtained anhydrosugar alcohol distillate was 97.5%.

The obtained distillate was placed in a jacketed reaction bath and 300 g of acetone (Samjeon Pure Chemical) was added thereto, and the crystallization was carried out in cooling the mixture to 0°C. After the crystallization was finished and then dehydration was conducted, the anhydrosugar alcohol crystals were separated from the mother liquid and recovered.

The obtained crystals were dissolved by adding distilled water thereto, and a solution with solid content of 37% was prepared. The prepared solution was decolorized by passing it through a column packed with fine granular active carbon having average particle size of 0.25 mm at the rate of 1.0 BV/h (bed volume/hour), and the decolorized anhydrosugar alcohol was then passed through a column packed with H-form strong cation exchange resin (TRILITE-SCR-BH, Samyang Corporation) at the rate of 1.5 BV/h, and the resulting liquid was then passed through a column packed with Cl-form strong anion exchange resin (TRILITE AMP24, Samyang Corporation) at the rate of 1.5 BV/h, to obtain the finally purified anhydrosugar alcohol.

### Example 1

1,000 g of aqueous solution of 40% by weight of the purified anhydrosugar alcohol obtained in the above Preparation Example was fed into a 2L beaker, and thereto 0.8mg of 2,2,6,6-tetramethylpiperidine (TMP) (20ppm based on the weight of anhydrosugar alcohol) was added and agitated for 5 minutes. After the agitation was finished, the solution was concentrated by using a rotary evaporator with 2L capacity. The concentration was conducted with adjusting the temperature to 100°C and the vacuum degree to 20 mmHg (= 2.66644 kPa) for 2 hours. The water content of the concentrated anhydrosugar alcohol measured by Karl Fischer method was 0.3% by weight. The concentrated anhydrosugar alcohol was diluted as an aqueous solution of 20% by weight concentration, and the UV transmittance thereof measured by using 5cm quartz cell at 275nm was 95%, and the pH of the aqueous solution of 20% by weight concentration measured at room temperature (25±3°C) was 6.5.

### Example 2

1,000 g of aqueous solution of 40% by weight of the purified anhydrosugar alcohol obtained in the above Preparation Example was fed into a 2L beaker, and thereto 1.6mg of 1,5-diazabicyclo[4.3.0]non-5-ene (DBN) (40ppm based on the weight of anhydrosugar alcohol) was added and agitated for 5 minutes. After the agitation was finished, the solution was concentrated by using a rotary evaporator with 2L capacity. The concentration was conducted with adjusting the temperature to 100°C and the vacuum degree to 20 mmHg (= 2.66644 kPa) for 2 hours. The water content of the concentrated anhydrosugar alcohol measured by Karl Fischer method was 0.3% by weight. The concentrated anhydrosugar alcohol was diluted as an aqueous solution of 20% by weight concentration, and the UV transmittance thereof measured by using 5cm quartz cell at 275nm was 94%, and the pH of the aqueous solution of 20% by weight concentration measured at room temperature was 7.4.

### Example 3

1,000 g of aqueous solution of 40% by weight of the purified anhydrosugar alcohol obtained in the above Preparation Example was fed into a 2L beaker, and thereto 0.4mg of 1,8-diazabicycloundec-7-ene (DBU) (10ppm based on the weight of anhydrosugar alcohol) was added and agitated for 5 minutes. After the agitation was finished, the solution was concentrated by using a rotary evaporator with 2L capacity. The concentration was conducted with adjusting the temperature to 100°C and the vacuum degree to 20 mmHg (= 2.66644 kPa) for 1 hour. The water content of the concentrated anhydrosugar alcohol measured by Karl Fischer method was 0.3% by weight. The concentrated anhydrosugar alcohol was diluted as an aqueous solution of 20% by weight concentration, and the UV transmittance thereof measured by using 5cm quartz cell at 275nm was 96%, and the pH of the aqueous solution of 20% by weight concentration measured at room temperature was 7.0.

### Comparative Example 1

1,000 g of aqueous solution of 40% by weight of the purified anhydrosugar alcohol obtained in the above Preparation Example was fed into a 2L beaker, and thereto 2.0mg of butylated hydroxy toluene (BHT) was added and agitated for 5 minutes. After the agitation was finished, the solution was concentrated by using a rotary evaporator with 2L capacity. The concentration was conducted with adjusting the temperature to 100°C and the vacuum degree to 20 mmHg (= 2.66644 kPa) for 2 hours. The water content of the concentrated anhydrosugar alcohol measured by Karl Fischer method was 0.3% by weight. The concentrated anhydrosugar alcohol was diluted as an aqueous solution of 20% by weight concentration, and the UV transmittance thereof measured by using 5cm quartz cell at 275nm was 82%, and the pH of the aqueous solution of 20% by weight concentration measured at room temperature was 6.2.

### Comparative Example 2

1,000 g of aqueous solution of 40% by weight of the purified anhydrosugar alcohol obtained in the above Preparation Example was concentrated by using a rotary evaporator with 2L capacity. The concentration was conducted with adjusting the temperature to 100°C and the vacuum degree to 20 mmHg (= 2.66644 kPa) for 2 hours. The water content of the concentrated anhydrosugar alcohol measured by Karl Fischer method was 0.3% by weight. The concentrated anhydrosugar alcohol was diluted as an aqueous solution of 20% by weight concentration, and the UV transmittance thereof measured by using 5cm quartz cell at 275nm was 88%, and the pH of the aqueous solution of 20% by weight concentration measured at room temperature was 5.4.

## Claims

1. A composition for preparing concentrated anhydrosugar alcohol, comprising anhydrosugar alcohol, and amine compound as additive for stabilization, wherein
the anhydrosugar alcohol is dianhydrohexitol,
the amine compound is selected from piperidine, 1,8-diazabicycloundec-7-ene, 1,5-diazabicyclo[4.3.0]non-5-ene and combinations thereof where the amine compound is unsubstituted or substituted by one or more substituents selected from linear or branched C₁-C₁₀alkyl, C₃-C₁₀cycloalkyl, aminoC₁-C₁₀alkyl, mono- or di-C₁-C₁₀alkylaminoC₁-C₁₀alkyl, carboxamide and amino,
the amount of the amine compound is 1 ppm or more and 300 ppm or less, based on the weight of the anhydrosugar alcohol, and
wherein the concentrated anhydrosugar alcohol shows a water content of less than 1% by weight, and a transmittance of 90% or higher to ultraviolet (UV) light with 275 nm wavelength when diluted as an aqueous solution of 20% by weight concentration.

2. The composition for preparing concentrated anhydrosugar alcohol of claim 1, wherein the amine compound has a pKa of 8 to 15.

3. The composition for preparing concentrated anhydrosugar alcohol of claim 1 or 2, which shows a pH of 6.5 to 7.5 when diluted as an aqueous solution of 20% by weight concentration.

4. The composition for preparing concentrated anhydrosugar alcohol of claim 1 or 2, which is in liquid or solid form.

5. A method for concentrating anhydrosugar alcohol, wherein the concentration of anhydrosugar alcohol is conducted in the presence of amine compound as additive for stabilization, wherein
the anhydrosugar alcohol is dianhydrohexitol,
the amine compound is selected from piperidine, 1,8-diazabicycloundec-7-ene, 1,5-diazabicyclo[4.3.0]non-5-ene and combinations thereof where the amine compound is unsubstituted or substituted by one or more substituents selected from linear or branched C₁-C₁₀alkyl, C₃-C₁₀cycloalkyl, aminoC₁-C₁₀alkyl, mono- or di-C₁-C₁₀alkylaminoC₁-C₁₀alkyl, carboxamide and amino,
the amount of the amine compound is 1 ppm or more and 300 ppm or less, based on the weight of the anhydrosugar alcohol,
wherein the concentration is conducted at a temperature of 90°C to 110°C under a pressure condition of 10 mmHg (= 1.33322 kPa) to 100 mmHg (= 13.3322 kPa) for 30 minutes or longer, and
wherein the anhydrosugar alcohol concentrated by the method shows a water content of less than 1% by weight, and a transmittance of 90% or higher to ultraviolet (UV) light with 275 nm wavelength when diluted as an aqueous solution of 20% by weight concentration.

6. The method for concentrating anhydrosugar alcohol of claim 5, wherein the anhydrosugar alcohol concentrated is that obtained by distilling a dehydration reaction product liquid resulting from dehydration reaction of hydrogenated sugar, and then purifying the distillation product.

7. The method for concentrating anhydrosugar alcohol of claim 6, wherein an acid catalyst is used in the dehydration reaction of hydrogenated sugar, and/or
wherein the distillation is conducted by using a thin-film evaporator, and/or
wherein the purification is conducted by one or more processes selected from crystallization, decolorization, cation exchange resin treatment or anion exchange resin treatment.

8. The method for concentrating anhydrosugar alcohol of claim 6, wherein in the purification, crystallization of the distillation product, decolorization of the crystallization product, and cation exchange resin treatment followed by anion exchange resin treatment of the decolorization product are conducted subsequently.

## Patentansprüche

1. Zusammensetzung zur Herstellung von konzentriertem Anhydrozuckeralkohol, umfassend Anhydrozuckeralkohol und eine Aminverbindung als Zusatzstoff zur Stabilisierung, wobei
der Anhydrozuckeralkohol Dianhydrohexitol ist,
die Aminverbindung ausgewählt ist aus Piperidin, 1,8-Diazabicycloundec-7-en, 1,5-Diazabicyclo[4.3.0]non-5-en und Kombinationen davon, wobei die Aminverbindung unsubstituiert oder durch einen oder mehrere Substituenten ausgewählt aus linearem oder verzweigtem C1-C10-Alkyl, C3-C10-Cycloalkyl, Amino-C1-C10-alkyl, Mono- oder Di-C1-C10-alkylamino-C1-C10-alkyl, Carboxamid und Amino substituiert ist,
die Menge der Aminverbindung, 1 ppm oder mehr und 300 ppm oder weniger beträgt, bezogen auf das Gewicht des Anhydrozuckeralkohols, und
wobei der konzentrierte Anhydrozuckeralkohol einen Wassergehalt von weniger als 1 Gew.-% und, wenn als wässrige Lösung mit einer Konzentration von 20 Gew.-% verdünnt, eine Transmission von 90 % oder höher für ultraviolettes (UV-)Licht mit einer Wellenlänge von 275 nm zeigt.

2. Zusammensetzung zur Herstellung von konzentriertem Anhydrozuckeralkohol nach Anspruch 1, wobei die Aminverbindung einen pKa von 8 bis 15 aufweist.

3. Zusammensetzung zur Herstellung von konzentriertem Anhydrozuckeralkohol nach Anspruch 1 oder 2, die, wenn als wässrige Lösung mit einer Konzentration von 20 Gew.-% verdünnt, einen pH von 6,5 bis 7,5 zeigt.

4. Zusammensetzung zur Herstellung von konzentriertem Anhydrozuckeralkohol nach Anspruch 1 oder 2, die in flüssiger oder fester Form vorliegt.

5. Anhydrozuckeralkoholkonzentrationsverfahren, wobei die Konzentration von Anhydrozuckeralkohol in Gegenwart einer Aminverbindung als Zusatzstoff zur Stabilisierung durchgeführt wird, wobei
der Anhydrozuckeralkohol Dianhydrohexitol ist,
die Aminverbindung ausgewählt ist aus Piperidin, 1,8-Diazabicycloundec-7-en, 1,5-Diazabicyclo[4.3.0]non-5-en und Kombinationen davon, wobei die Aminverbindung unsubstituiert oder durch einen oder mehrere Substituenten ausgewählt aus linearem oder verzweigtem C1-C10-Alkyl, C3-C10-Cycloalkyl, Amino-C1-C10-alkyl, Mono- oder Di-C1-C10-alkylamino-C1-C10-alkyl, Carboxamid und Amino substituiert ist,
die Menge der Aminverbindung 1 ppm oder mehr und 300 ppm oder weniger beträgt, bezogen auf das Gewicht des Anhydrozuckeralkohols,
wobei die Konzentration bei einer Temperatur von 90 °C bis 110 °C unter einer Druckbedingung von 10 mmHg (= 1,33322 kPa) bis 100 mmHg (= 13,3322 kPa) für 30 Minuten oder länger durchgeführt wird, und
wobei der durch das Verfahren konzentrierte Anhydrozuckeralkohol einen Wassergehalt von weniger als 1 Gew.-% und, wenn als wässrige Lösung mit einer Konzentration von 20 Gew.-% verdünnt, eine Transmission von 90 % oder höher für ultraviolettes (UV-)Licht mit einer Wellenlänge von 275 nm zeigt.

6. Anhydrozuckeralkoholkonzentrationsverfahren nach Anspruch 5, wobei der konzentrierte Anhydrozuckeralkohol derjenige ist, der durch Destillieren einer Dehydratisierungsreaktionsproduktflüssigkeit, die aus einer Dehydratisierungsreaktion von hydriertem Zucker resultiert, und anschließendes Reinigen des Destillationsprodukts erhalten wird.

7. Anhydrozuckeralkoholkonzentrationsverfahren nach Anspruch 6, wobei bei der Dehydratisierungsreaktion von hydriertem Zucker ein Säurekatalysator verwendet wird, und/oder
wobei die Destillation unter Verwendung eines Dünnschichtverdampfers durchgeführt wird, und/oder
wobei die Reinigung durch ein oder mehrere Verfahren ausgewählt aus Kristallisation, Entfärbung, Kationenaustauscherharzbehandlung oder Anionenaustauscherharzbehandlung durchgeführt wird.

8. Anhydrozuckeralkoholkonzentrationsverfahren nach Anspruch 6, wobei bei der Reinigung eine Kristallisation des Destillationsprodukts, eine Entfärbung des Kristallisationsprodukts und eine Kationenaustauscherharzbehandlung, gefolgt von einer Anionenaustauscherharzbehandlung des Entfärbungsprodukts, nacheinander durchgeführt werden.

## Revendications

1. Une composition pour la production de polyol anhydre concentré, comprenant un polyol anhydre et un composé aminé en tant qu'additif pour la stabilisation, dans laquelle
le polyol anhydre est un dianhydrohexitol,
le composé aminé est sélectionné parmi la pipéridine, le 1,8-diazabicycloundéc-7-ène, le 1,5-diazabicyclo[4.3.0]non-5-ène et des combinaisons de ceux-ci, le composé aminé étant non substitué ou substitué par un ou plusieurs substituants sélectionnés parmi un alkyle en C₁-C₁₀ linéaire ou ramifié, un cycloalkyle en C₃-C₁₀, un aminoalkyle en C₁-C₁₀, un mono- ou di-alkyl(C₁-C₁₀)aminoalkyle en C₁-C₁₀, un carboxamide et un amino,
la quantité du composé aminé est de 1 ppm ou plus et de 300 ppm ou moins, sur la base du poids du polyol anhydre, et
dans laquelle le polyol anhydre concentré présente une teneur en eau inférieure à 1 % en poids et, lorsqu'il est dilué sous forme d'une solution aqueuse ayant une concentration de 20 % en poids, une transmittance de 90 % ou plus pour une lumière ultraviolette (UV) ayant une longueur d'onde de 275 nm.

2. La composition pour la production de polyol anhydre concentré selon la revendication 1, dans laquelle le composé aminé a un pKa de 8 à 15.

3. La composition pour la production de polyol anhydre concentré selon la revendication 1 ou 2, qui, lorsqu'elle est diluée sous forme d'une solution aqueuse ayant une concentration de 20 % en poids, présente un pH de 6,5 à 7,5.

4. La composition pour la production de polyol anhydre concentré selon la revendication 1 ou 2, qui est sous forme liquide ou solide.

5. Un procédé de concentration de polyol anhydre, dans lequel la concentration de polyol anhydre est effectuée en présence d'un composé aminé en tant qu'additif pour la stabilisation, dans lequel
le polyol anhydre est un dianhydrohexitol,
le composé aminé est sélectionné parmi la pipéridine, le 1,8-diazabicycloundéc-7-ène, le 1,5-diazabicyclo[4.3.0]non-5-ène et des combinaisons de ceux-ci, le composé aminé étant non substitué ou substitué par un ou plusieurs substituants sélectionnés parmi un alkyle en C₁-C₁₀ linéaire ou ramifié, un cycloalkyle en C₃-C₁₀, un aminoalkyle en C₁-C₁₀, un mono- ou di-alkyl(C₁-C₁₀)aminoalkyle en C₁-C₁₀, un carboxamide et un amino,
la quantité du composé aminé est de 1 ppm ou plus et de 300 ppm ou moins, sur la base du poids du polyol anhydre,
dans lequel la concentration est effectuée à une température de 90 °C à 110 °C sous une condition de pression de 10 mmHg (= 1,33322 kPa) à 100 mmHg (= 13,3322 kPa) pendant 30 minutes ou plus, et
dans lequel le polyol anhydre concentré par le procédé présente une teneur en eau inférieure à 1 % en poids et, lorsqu'il est dilué sous forme d'une solution aqueuse ayant une concentration de 20 % en poids, une transmittance de 90 % ou plus pour une lumière ultraviolette (UV) ayant une longueur d'onde de 275 nm.

6. Le procédé de concentration de polyol anhydre selon la revendication 5, dans lequel le polyol anhydre concentré est celui obtenu en distillant un liquide de produit de réaction de déshydratation résultant d'une réaction de déshydratation d'un sucre hydrogéné, et puis en purifiant le produit de distillation.

7. Le procédé de concentration de polyol anhydre selon la revendication 6, dans lequel un catalyseur acide est utilisé dans la réaction de déshydratation du sucre hydrogéné, et/ou
dans lequel la distillation est effectuée en utilisant un évaporateur à film mince, et/ou
dans lequel la purification est effectuée par un ou plusieurs procédés sélectionnés parmi une cristallisation, une décoloration, un traitement par résine échangeuse de cations ou un traitement par résine échangeuse d'anions.

8. Le procédé de concentration de polyol anhydre selon la revendication 6, dans lequel, lors de la purification, une cristallisation du produit de distillation, une décoloration du produit de cristallisation et un traitement par résine échangeuse de cations suivi d'un traitement par résine échangeuse d'anions du produit de décoloration sont effectués successivement.
